# EUROPÄISCHE PATENTSCHRIFT

(11) **EP 0 688 556 B1**
(45) Veröffentlichungstag und Bekanntmachung des Hinweises auf die Patenterteilung: **03.09.1997**
(21) Anmeldenummer: 95107270.1
(22) Anmeldetag: 12.05.1995
(51) Int. Cl.: A61K 7/11

(54) **Haarspray**
Hair spray formulation
Laque aérosol capillaire

(30) Priorität: 20.06.1994 DE 4421562
(43) Veröffentlichungstag der Anmeldung: 27.12.1995
(73) Patentinhaber: GOLDWELL GmbH, D-64297 Darmstadt (DE)
(72) Erfinder: Bünning, Einhard, D-64342 Seeheim-Jugenheim (DE); Cajan, Christine, D-56130 Bad Ems (DE)

(56) Entgegenhaltungen:
- EP-A- 0 205 306
- EP-A- 0 431 219
- WO-A-90/00045
- WO-A-93/03703
- US-A- 4 834 968
- US-A- 4 839 167

## Beschreibung

Die vorliegende Erfindung betrifft ein Haarspray mit einem niedrigen Gehalt an flüchtigen Bestandteilen, das verbesserte Gebrauchseigenschaften aufweist.

Haarsprays mit einem geringen Anteil an flüchtigen organischen Bestandteilen (in der Fachwelt als "VOC", volatile organic compounds, bekannt) sind aus Umweltschutzgründen in jüngerer Zeit populär geworden.

Typische derartige Zusammensetzungen sind beispielsweise in der EP-A 557 087 beschrieben.

Der, aus Kosten- und Umweltgründen, beliebteste Ersatzstoff für diese flüchtigen Bestandteile ist Wasser.

Es hat sich jedoch gezeigt, daß diese Möglichkeit dadurch begrenzt wird, daß der Sprühstrahl beim Austritt aus der Dose eine deutliche Tröpfchenbildung erkennen läßt, und dadurch nach dem Verdunsten der flüssigen Anteile auf dem trockenen Haar perlartige Harzrückstände des Filmbildners auf dem Haar erkennbar sind, also keine gleichmäßige Filmbildung stattfindet.

Die vorliegende Erfindung geht von der Aufgabenstellung aus, diese Nachteile zu überwinden und einen Haarspray mit einem geringen Anteil flüchtiger Bestandteile zur Verfügung zu stellen, der ein verbessertes Sprühverhalten zeigt, keine Tröpfchenbildung begünstigt, eine gleichmäßige Verteilung des Filmbildners auf dem Haar bewirkt und auch eine verkürzte Trocknungszeit nach der Applikation auf dem Haar aufweist.

Diese Aufgabe wird durch die Verwendung eines Haarsprays gelöst, das
a) etwa 5 bis etwa 50, vorzugsweise 15 bis 40 Gew.-% mindestens eines niederen Alkohols aus der Gruppe Ethanol, n-Propanol und/oder Isopropylalkohol;
b) etwa 15 bis etwa 70, vorzugsweise etwa 20 bis etwa 50 Gew.-% Wasser;
c) etwa 1 bis etwa 15, vorzugsweise etwa etwa 2,5 bis etwa 10 Gew.-% mindestens eines filmbildenden Polymeren; und
d) etwa 0,1 bis etwa 3, vorzugsweise etwa 0,5 bis etwa 1,5 Gew.-% eines wasserdispergierbaren Silikon-Wachses der allgemeinen Formel I 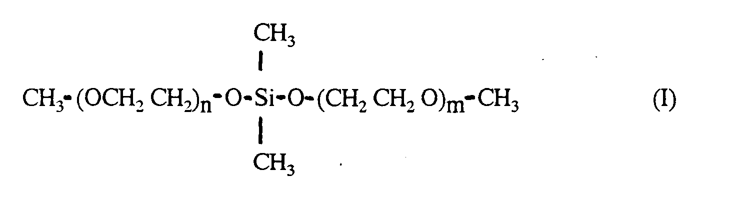 worin n und m gleich oder verschieden sein können und eine ganze Zahl zwischen 10 und 20, vorzugsweise zwischen 14 und 18, bedeuten, enthält,
   und entweder als manuell zu betätigende Pumpsprayzusammensetzung oder mit Dimethylether als Treibgas oder einem komprimierten Gas aus der Gruppe Distickstoffoxid, Stickstoff, Luft und/oder Kohlendioxid konfektionierte Aerosolsprühzusammensetzung vorliegt.

Der bevorzugte Anteil von Dimethylether in Aerosolsprays liegt zwischen etwa 10 und etwa 40, vorzugsweise etwa 20 bis 30 Gew.-% der Gesamtzusammensetzung.

Ein Teil des Dimethylethers kann durch Propan, Butan und/oder Isobutan ersetzt sein, die beispielsweise bis zu etwa 30% des Gesamttreibgas-Gemisches ausmachen können.

Falls komprimierte Gase eingesetzt werden, wird deren Menge so gewählt, daß die Aerosolzusammensetzung einen Druck von etwa 5 bis etwa 15, vorzugsweise 6 bis 14, insbesondere 8 bis 12 bar aufweist.

Es ist zweckmäßig, den erfindungsgemäßen Haarspray-Zusammensetzungen zur weiteren Optimierung des Sprühbildes und als Lösungsmittel Alkane mit 5 bis 9 Kohlenstoffatomen zuzusetzen.
Solche sind insbesondere n-Pentan, iso-Pentan, n-Hexan, iso-Hexan, n-Heptan, iso-Heptan, n-Octan, iso-Octan und/oder n-Nonan.
Der Zusatz dieser Alkane zu Aerosolsprays ist an sich aus den WO-A 91/08730 und 93/18735 bekannt; die optimale Menge liegt bei etwa 5 bis etwa 25 Gew.-% der Gesamtzusammensetzung.

Es wird angenommen, daß der Zusatz des in der allgemeinen Formel I definierten wasserdispergierbaren Silikon-Wachses die Spreitwirkung des Gesamtproduktes verbessert und dadurch die verbesserten Sprüh- und Filmbildungseigenschaften des Produktes bewirkt.

Diese Wirkung kann noch dadurch verbessert werden, daß das oben definierte Silikon-Wachs im Gemisch mit einem Polyethlenglykoldimethylether mit etwa der gleichen Anzahl EO-Gruppen im Verhältnis von etwa 75:25 bis 90:10, vorzugsweise etwa 80:20 bis etwa 85:15, vorliegt.

Ein entsprechendes Handelsprodukt ist beispielsweise unter der Bezeichnung "DOW CORNING^{R}2501 COSMETIC WAX" im Handel und wird dort vor allem zur Anwendung als Feuchthaltemittel empfohlen.

Als Filmbildner sind in den erfindungsgemäßen Haarsprays grundsätzlich alle für diesen Zweck vorgeschlagenen und bekannten Polymeren geeignet.

Einsetzbare anionische Polymere sind insbesondere die bekannten Crotonsäure- und Acrylsäure-Copolymeren, beispielsweise Vinylacetat/Crotonsäure- oder Vinylacetat/Vinylneodecanoat/Crotonsäur-Copolymere des Typs "Resyn^{R}"; Natriumacrylat/Vinylalkohol-Copolymere des Typs "Hydagen^{R}F"; Natriumpolystyrolsulfonat, z.B. "Flexan^{R}130"; Ethylacrylat/Acrylsäure/N-tert.--Butylacrylamid-Copolymere des Typs "Ultrahold^{R}"; Vinylpyrrolidon/Vinylacetat/Itaconsäure-Copolymere; Acrylsäure/Acrylamid-Copolymere bzw. Natriumsalze derselben vom Typ "Reten^{R}_{"}, etc.

Weitere geeignete anionische Polymere sind Vinylalkylether-, insbesondere Methylvinylether/Maleinsäure-Copolymere, die durch Hydrolyse von Vinylether/Maleinsäureanhydrid-Copolymeren entstehen und unter der Handelsbezeichnung "Gantrez^{R} AN oder ES" vertrieben werden. Diese Polymeren können auch teilverestert sein, beispielsweise "Gantrez^{R} ES 225", der Ethylester eines Ethylvinylether/Maleinsäure-Copolymers, oder der Butyl- oder Isobutylester desselben.

Geeignete nichtionische Polymere, die in den erfindungsgemäßen Haarsprays alleine oder vorzugsweise auch im Gemisch mit anionischen und/oder amphoteren bzw. zwitterionischen Polymeren verwendet werden, sind insbesondere Vinylpyrrolidon-Homo- oder -Copolymerisate, beispielsweise solche vom Typ "Luviskol^{R}".

Dies sind Polyvinylpyrrolidon selbst, Copolymerisate aus Vinylpyrrolidon und Vinylacetat, sowie Terpolymere aus Vinylpyrrolidon, Vinylacetat und weiteren Vinylestern, z.B. Vinylpropionat.

Weitere Copolymere sind solche aus Vinylpyrrolidon und Dimethylaminoethylmethacrylat (Copolymer 845, 937 bzw. 958 der GAF Co.) und Terpolymere aus Vinylcaprolactam, Vinylpyrrolidon und Dimethylaminoethylmethacrylat (Copolymer VC-713 der GAF Co.).

Auch Filmbildner auf natürlicher Basis, z.B. Chitosan und dessen Derivate, können eingesetzt werden, insbesondere im Gemisch mit synthetischen Polymeren.

Als amphotere Polymere, die entweder alternativ zu oder im Gemisch mit den anionischen und/oder nichtionischen Polymeren zu Einsatz gelangen, seien insbesondere Copolymerisate aus N-Octylacrylamid, (Meth)Acrylsäure und tert.-Butylaminoethylmethacrylat vom Typ "Amphomer^{R}"; Copolymerisate aus Methacryloylethylbetain und Alkylmethacrylaten vom Typ "Yukaformer^{R}", z.B. das Butylmethacrylat-Copolymere "Yukaformer^{R} Am75"; Copolymerisate aus Carboxylgruppen oder Sulfongruppen enthaltenden Monomeren, z.B. (Meth)Acrylsäure und Itaconsäure, mit basische, insbesondere Aminogruppen enthaltenden Monomeren wie Mono- bzw. Dialkylaminoalkyl(meth)acrylaten bzw. Mono- bzw. Dialkylaminoalkyl(meth)acrylamiden; Copolymere aus N-Octylacrylamid, Methylmethacrylat, Hydroxypropylmethacrylat, N-tert.-Butylaminoethylmethacrylat und Acrylsäure sowie die aus der US-A 3,927,199 bekannten Copolymeren genannt.

Es können selbstverständlich, wie auch bei den anionischen Polymeren, alle in Haarpflegemitteln einsetzbaren amphoteren Polymeren erfindungsgemäß eingesetzt werden.

Die anionischen, nichtionischen und amphoteren Polymeren können, wie bereits ausgeführt, auch im Gemisch untereinander verwendet werden.

Hierbei haben sich insbesondere Gemische aus dem Handelsprodukt "Amphomer^{R}", einem Copolymerisat aus N-Octylacrylamid, Acrylsäure und tert.-Butylaminoethylmethacrylat einerseits und Vinylacetat/Crotonsäure-Polymerisaten des Typs "Resyn^{R}" andererseits besonders bewährt, jedoch können,wie bereits erwähnt, generell Mischungen aus einem oder mehreren der oben jeweils aufgeführten anionischen, nichtionischen und amphoteren Polymeren eingesetzt werden.

Die erfindungsgemäße Zusammensetzungen können die in Haarsprays üblichen Zusätze enthalten, beispielsweise weitere Lösungsmittel wie niedere Polyalkohole und deren toxikologisch verträglichen Ether und Ester, Weichmacher, leicht- und schwerflüchtige Silikone, Neutralisationsmittel für carboxylgruppenhaltige Filmbildner, Antistatika, UV-Absorber, Parfums, etc., wie es aus dem Stand der Technik bekannt ist.

Die folgenden Beispiele dienen der näheren Illustration der Erfindung.

### Beispiel 1

| | |
|---|---|
| Ethanol | 34,7 (Gew.-%) |
| Acrylsäure/Ethylacrylat/N-tert.-Butylacrylamid-Terpolymer | 8,0 |
| 2-Amino-2-methyl-1-propanol | 0,6 |
| Wasserdispergierbares Silikon-Wachs entsprechend Formel I (n=m=16) | 0,5 |
| Parfum | 0,2 |
| n-Pentan | 8,0 |
| Dimethylether | 28,0 |
| Wasser | ad 100,0 |

Es wird ein ausgezeichnet festigender Haarspray mit homogenem Sprühstrahl erhalten, der nach Applikation auf dem Haar keinerlei sichtbaren teilchenförmigen Harzrückstände ergibt.

### Beispiel 2

| | |
|---|---|
| Ethanol | 26,6 (Gew.-%) |
| Acrylsäure/Ethylacrylat/N-tert. Butylacrylamid-Terpolymer | 5,0 |
| Vinylpyrrolidon/Vinylacetat (30:70) -Copolymerisat (50%-ig in Isopropylalkohol) | 6,0 |
| 2-Amino-2-methyl-1-propanol | 0,4 |
| Wasserdispergierbares Silikon-Wachs entsprechend Formel I (n=m=16) | 0,8 |
| Polyethylenglykol-(16)-dimethylether | 0,2 |
| Parfum | 0,2 |
| n-Pentan | 8,0 |
| Dimethylether | 28,0 |
| Wasser | ad 100,0 |

Es wird ein Haarspray mit Sprüheigenschaften erthalten, die denjenigen nach Beispiel 1 entsprechen, und der darüber hinaus excellente Festigungseigenschaften aufweist.

### Beispiel 3

| | |
|---|---|
| Ethanol | 31,9 (Gew.-%) |
| Acrylsäure/Ethylacrylat/ N-tert Butylacrylamid-Terpolymer Vinylpyrrolidon/Vinylacetat/ Vinylpropionat | 5,0 |
| (30:40:30)-Terpolymer (50%-ig in Isopropylalkohol) | 6,0 |
| 1-Amino-2-methyl-1-propanol | 0,4 |
| Wasserdispergierbares Silikon-Wachs entsprechend Formel I (n=m=16) | 0,5 |
| Polyethylenglykol-(16)-dimethylether | 0,1 |
| Parfum | 0,2 |
| n-Butan | 6,0 |
| Dimethylether | 30,0 |
| Wasser | ad 100,0 |

Die Sprüh- und Festigungseigenschaften dieses Haarsprays entsprechen im wesentlichen denjenigen des Haarsprays nach Beispiel 2.

## Patentansprüche

1. Haarspray mit einem geringen Anteil an flüchtigen Bestandteilen, das
a) etwa 5 bis etwa 50 Gew.-% mindestens eines niederen Alkohols aus der Gruppe Ethanol, n-Propanol und/oder Isopropylalkohol;
b) etwa 15 bis etwa 70 Gew.-% Wasser;
c) etwa 1 bis etwa 15 Gew.-% mindestens eines filmbildenden Polymeren;
d) etwa 0,1 bis etwa 3 Gew.-% eines wasserdispergierbaren Silikon-Wachses der allgemeinen Formel I 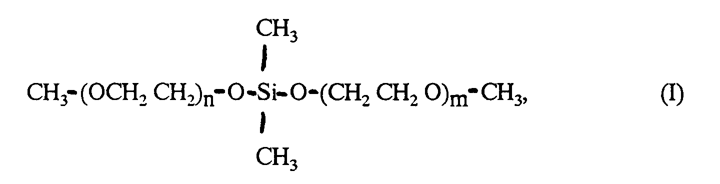 worin n und m gleich oder verschieden sein können und eine ganze Zahl zwischen 10 und 20 bedeuten,
enthält, wobei sich die angegebenen Prozentsätze auf die Gesamtzusammensetzung des Haarsprays beziehen,
und entweder
e1) als mit Dimethylether als Treibgas oder einem komprimierten Gas aus der Gruppe Distickstoffoxid, Luft, Stickstoff und/oder Kohlendioxid konfektionierte Aerosolsprühzusammensetzung, oder
e2) als manuell zu betätigender Pumpspray vorliegt.

2. Haarspray nach Anspruch 1, dadurch gekennzeichnet, daß das wasserdispergierbare Silikon-Wachs im Gemisch mit einem Polyethylenglykoldimethylether im Gewichtsverhältnis 90:10 bis 75:25 vorliegt.

3. Haarspray nach Anspruch 1 oder 2, dadurch gekennzeichnet, daß es etwa 2,5 bis etwa 10 Gew.-% des filmbildenden Polymeren enthält.

4. Haarspray nach einem der Ansprüche 1 bis 3, dadurch gekennzeichnet, daß es als filmbildendes Polymer mindestens ein anionisches, amphoteres und/oder nichtionisches Polymer enthält.

5. Haarspray nach Anspruch 4, dadurch gekennzeichnet, daß es als nichtionisches Polymer ein Vinylpyrrolidon-Homo- oder -Copolymerisat enthält.

6. Haarspray nach Anspruch 4 dadurch gekennzeichnet, daß es als amphoteres Polymer ein N-Octyl(meth)acrylamid/tert.-Butylaminoethylmethacrylat/Acrylsäure-Terpolymerisat enthält.

7. Haarspray nach Anspruch 4, dadurch gekennzeichnet, daß es als anionisches Polymer ein Crotonsäure-Copolymerisat enthält.

8. Haarspray nach Anspruch 4, dadurch gekennzeichnet, daß es als anionisches Polymer ein Acrylsäure-Copolymerisat enthält.

## Claims

1. Hair spray having a low proportion of volatile compounds comprising
a) about 5% to about 50% by wt. of at least one lower alcohol selected from the group of ethanol, n-propanol and (or) isopropyl alcohol;
b) about 15% to about 70% by wt. water;
c) about 1% to about 15% by wt. of at least one film-forming polymer;
d) about 0.1% to about 3% by wt. of a water-dispersible silicone wax of the general formula I 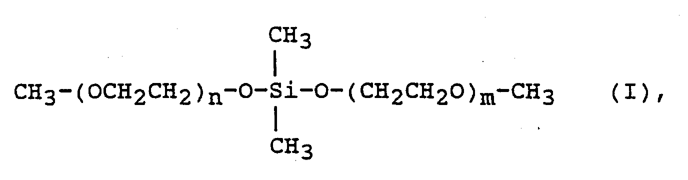 wherein n and m may be identical or different and denote an integer between 10 and 20, all percentages calculated to the total hair spray composition; and either packed
e1) as an aerosol spray composition comprising dimethyl ether as propellent or a compressed gas selected from the group of dinitrogen oxide, air, nitrogen and (or) carbon dioxide, or
e2) as a manually operated pump spray.

2. Hair spray according to claim 1, characterized in that the water-dispersible silicone wax is present in admixture with polyethylene glycol dimethyl ether in a weight proportion of 90 : 10 to 75 : 25.

3. Hair spray according to claim 1 or 2, characterized in that it comprises about 2.5% to about 10% by wt. of the film-forming polymer.

4. Hair spray according to one of the claims 1 to 3, characterized in that it comprises as film-forming polymer at least one anionic, amphoteric and (or) nonionic polymer.

5. Hair spray according to claim 4, characterized in that it comprises as nonionic polymer a vinyl pyrrolidone homo- or copolymer.

6. Hair spray according to claim 4, characterized in that it comprises as amphoteric polymer a N-octyl (meth)acrylamide/tert.-butyl aminoethyl methacrylate/acrylic acid terpolymer.

7. Hair spray according to claim 4, characterized in that it comprises as anionic polymer a crotonic acid copolymer.

8. Hair spray according to claim 4, characterized in that it comprises as anionic polymer an acrylic acid copolymer.

## Revendications

1. Laque capillaire ayant une faible proportion de composants volatiles, contenant
a) environ 5 à environ 50% en poids d'au moins un alcool inférieur choisi parmi le groupe constitué de l'éthanol, du n-propanol et/ou de l'alcool isopropylique;
b) environ 15 à environ 70% en poids d'eau;
c) environ 1 à environ 15% en poids d'au moins un polymère filmogène;
d) environ 0,1 à environ 3% en poids d'une cire de silicone dispersable dans l'eau de formule générale I 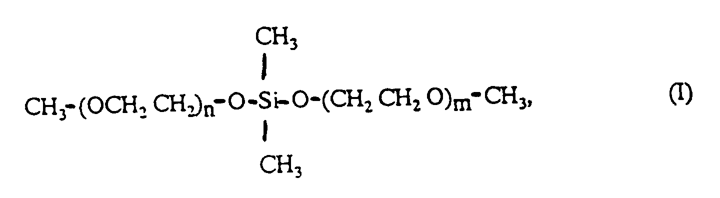 dans laquelle n et m peuvent être identiques ou différents et représentent un nombre entier compris entre 10 et 20,
les pourcentages indiqués étant relatifs à la composition totale de la laque capillaire,
et se présente soit
e1) sous la forme d'une composition d'aérosol à vaporiser préparée avec de l'éther diméthylique en tant que gaz propulseur ou un gaz comprimé choisi parmi le groupe constitué du dioxyde d'azote, de l'air, de l'azote et/ou du dioxyde de carbone, soit
e2) sous forme d'un vaporisateur à pompe à actionner manuellement.

2. Laque capillaire selon la revendication 1, caractérisée en ce que la cire de silicone dispersable dans l'eau est présente en mélange avec un polyéthylèneglycoldiméthyléther dans un rapport molaire de 90:10 à 75:25.

3. Laque capillaire selon la revendication 1 ou 2, caractérisée en ce qu'elle contient environ 2,5 à environ 10% en poids du polymère filmogène.

4. Laque capillaire selon l'une quelconque des revendications 1 à 3, caractérisée en ce qu'elle contient en tant que polymère filmogène au moins un polymère anionique, amphotère et/ou non ionique.

5. Laque capillaire selon la revendication 4, caractérisée en ce qu'elle contient en tant que polymère non ionique un homo- ou copolymère de la vinylpyrrolidone.

6. Laque capillaire selon la revendication 4, caractérisée en ce qu'elle contient en tant que polymère amphotère un terpolymère N-octyl(méth)acrylamide/méthacrylate de tertbutylaminoéthyle/acide acrylique.

7. Laque capillaire selon la revendication 4, caractérisée en ce qu'elle contient en tant que polymère anionique un copolymère de l'acide crotonique.

8. Laque capillaire selon la revendication 4, caractérisée en ce qu'elle contient en tant que polymère anionique un copolymère de l'acide acrylique.
